# FASCICULE DE BREVET EUROPEEN

(11) **EP 1 981 451 B1**
(45) Date de publication et mention de la délivrance du brevet: **08.01.2014**
(21) Numéro de dépôt: 07712649.8
(22) Date de dépôt: 15.01.2007
(51) Int. Cl.: A61F 5/00

(54) **ANNEAU GASTRIQUE**
MAGENBAND
GASTRIC RING

(30) Priorité: 16.01.2006 FR 0600375
(43) Date de publication de la demande: 22.10.2008
(73) Titulaire: SURGICAL-IOC, 42000 Saint-Etienne (FR)
(72) Inventeur: MOUTON, Didier, F-42270 Saint Priest en Jarez (FR); SODJI, Maxime, 8700 Limoges (FR); DE LA CRUZ VIGO, Felipe, 28040 Madrid (ES); DE LA CRUZ VIGO, José Luiz, 24008 Leon (ES)
(74) Mandataire: Myon, Gérard Jean-Pierre
(86) Numéro de dépôt international: PCT/FR2007/000066
(87) Numéro de publication internationale: WO 2007/080334

(56) Documents cités:
- EP-A- 1 036 545
- WO-A-02/096326
- FR-A- 2 799 118
- US-A- 4 592 355
- US-A- 5 462 542
- US-B1- 6 226 839

## Description

L'invention a trait à un anneau gastrique tel qu'utilisé pour les opérations de gastroplastie. Un tel anneau est prévu pour entourer une partie d'estomac afin d'assurer un effet de rétrécissement ou calibrage dans le cadre du traitement de l'obésité.

Il est connu, par exemple de FR-A-2 799 118, d'utiliser un anneau gastrique pourvu d'une poche dilatable qui est remplie de liquide physiologique en fonction du diamètre recherché pour le rétrécissement gastrique obtenu grâce à un tel anneau. Le contrôle du gonflage de la poche est relativement complexe et nécessite l'utilisation d'un boîtier sous-cutané, avec des risques de contamination ou de fuite.

WO-A-02/096326 permet de pallier ces inconvénients en proposant un anneau gastrique dépourvu de partie dilatable et destiné à venir en appui, par une partie courante ménagée entre ses extrémités, sur la paroi gastrique. Un tel anneau s'avère tout à fait efficace mais son diamètre intérieur en configuration fermée ne peut pas être ajusté. Or, en fonction de la morphologie du patient des différentes épaisseurs de parois gastriques, de ses habitudes alimentaires et de son profil psychologique, différents diamètres de rétrécissement ou de calibrage gastrique doivent pouvoir être prévus, l'anneau de l'art antérieur devant être prévu en plusieurs tailles.

Par ailleurs, il est connu de EP-A-1 036 545 de munir les bords d'une extrémité d'un anneau gastrique de reliefs d'accrochage en configuration fermée. Ces reliefs ne sont pas fiables et un décrochage ne peut pas être exclu.

C'est à ces inconvénients qu'entend plus particulièrement remédier l'invention en proposant un anneau gastrique ne nécessitant pas le contrôle d'une partie dilatable et dont le diamètre intérieur peut être modifié lors de sa pose pour l'adapter à différentes configurations d'implantation.

A cet effet, l'invention concerne un anneau gastrique dépourvu de partie dilatable et comprenant deux extrémités et, entre ces extrémités, une portion intermédiaire à section pleine destinée à venir en appui simple sur une partie d'estomac à entourer, une première extrémité de cet anneau formant une ouverture dans laquelle peut être introduite la deuxième extrémité, cet anneau comprenant des reliefs de blocage de la seconde extrémité dans l'ouverture précitée, dans au moins deux positions distinctes. Cet anneau est caractérisé en ce qu'il comprend, en plus de ces reliefs, des moyens de verrouillage de la deuxième extrémité dans l'ouverture, dans chacune des positions précitées, ces moyens de verrouillage comprenant eux-mêmes deux premiers orifices ménagés dans deux cloisons appartenant à la première extrémité et entre lesquelles est définie l'ouverture, et au moins deux deuxièmes orifices ménagés dans l'autre extrémité, l'un des deuxièmes orifices étant aligné avec les premiers orifices dans chacune des positions précitées, ces orifices formant alors ensemble un canal de passage d'un organe de retenue des extrémités de l'anneau l'une par rapport à l'autre, alors que les deux premiers orifices sont alignés selon une direction perpendiculaire à un axe longitudinal de l'ouverture.

Grâce à l'invention, le diamètre intérieur de l'anneau gastrique en configuration d'utilisation peut varier en fonction de la position dans laquelle sa deuxième extrémité est immobilisée dans l'ouverture de sa première extrémité. Un ajustement rapide et fiable du diamètre interne de l'anneau gastrique est ainsi possible.

Selon des aspects avantageux mais non obligatoires de l'invention, un tel anneau peut incorporer une ou plusieurs des caractéristiques suivantes :
- l'ouverture de la première extrémité est pourvue d'un relief de blocage apte à coopérer, en fonction du diamètre sélectionné pour l'anneau, avec l'un de plusieurs reliefs de blocage complémentaires qui sont ménagés sur la deuxième extrémité ;
- le relief de blocage de l'ouverture est une dent qui fait saillie à partir d'un bord de cette ouverture en direction du bord opposé ;
- les reliefs complémentaires ménagés sur la deuxième extrémité sont formés par des dents disposées l'une derrière l'autre selon un axe longitudinal de la deuxième extrémité ;
- les deuxièmes orifices sont ménagés respectivement au niveau des dents de la deuxième extrémité, dans une partie de la deuxième extrémité qui prolonge la portion intermédiaire, les orifices s'étendant selon des directions perpendiculaires à un axe longitudinal de la seconde extrémité ;
- les dents de l'ouverture et de la deuxième extrémité ont des surfaces inclinées, respectivement par rapport à un axe médian de l'ouverture et par rapport à l'axe longitudinal de la deuxième extrémité, dans un sens compatible avec le déplacement de la deuxième extrémité à travers l'ouverture correspondant à un resserrement de l'anneau ;
- les dents ont des surfaces perpendiculaires respectivement à un axe médian de l'ouverture et à l'axe longitudinal de la deuxième extrémité, ces faces étant en appui l'une contre l'autre lorsque la seconde extrémité est immobilisée dans l'ouverture de la première extrémité, dans l'une des positions précitées ;
- un volume défini entre deux dents adjacentes ménagées sur la deuxième extrémité est sensiblement complémentaire de la dent qui fait saillie à partir d'un bord de l'ouverture ;
- la portion intermédiaire de l'anneau est pourvue, sur chacun de ses bords longitudinaux, d'un orifice de passage d'un fil de ligature, ces orifices étant sensiblement diamétralement opposés lorsque la deuxième extrémité est immobilisée dans l'ouverture dans l'une des positions précitées.

L'invention sera mieux comprise et d'autres avantages de celle-ci apparaîtront plus clairement à la lumière de la description qui va suivre d'un mode de réalisation d'un anneau gastrique conforme à son principe, donnée uniquement à titre d'exemple et faite en référence aux dessins annexés dans lesquels :
- la figure 1 est une représentation schématique d'un anneau gastrique conforme à l'invention implanté sur un estomac ;
- la figure 2 est une vue en perspective de l'anneau de la figure 1 en configuration ouverte ;
- la figure 3 est une vue en perspective, selon un autre angle, de l'anneau de la figure 1 ;
- la figure 4 est une vue de côté de l'anneau dans la configuration des figures 1 et 3 ;
- la figure 5 est une vue de face de l'anneau dans la configuration des figures 1, 3 et 4 ;
- la figure 6 est une coupe selon la ligne VI-VI à la figure 5 ;
- la figure 7 est une coupe analogue à la figure 6 lorsque l'anneau est dans une autre configuration d'utilisation ;
- la figure 8 est une coupe analogue à la figure 6 lorsque l'anneau est dans encore une autre configuration d'utilisation et
- la figure 9 est une représentation schématique d'une autre configuration d'implantation de l'anneau des figures précédentes.

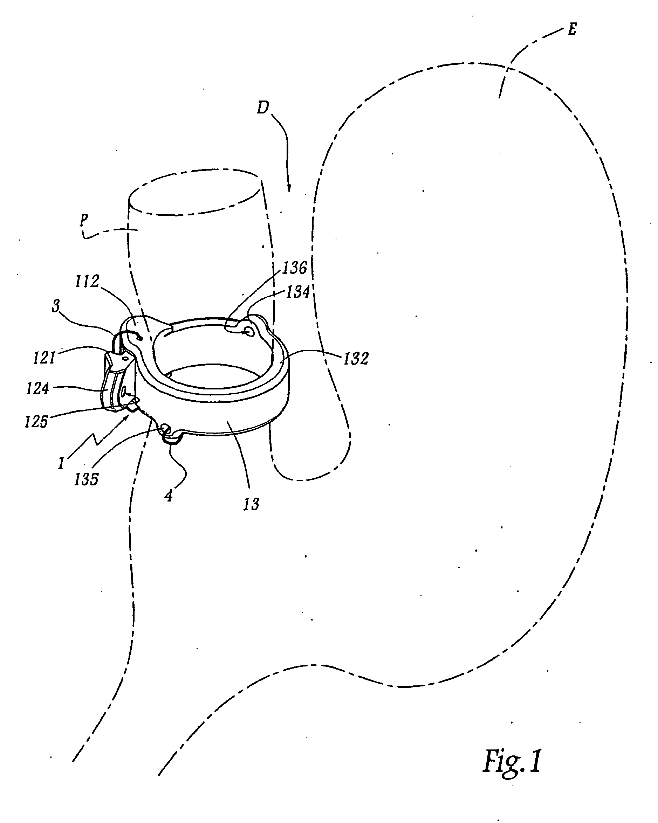

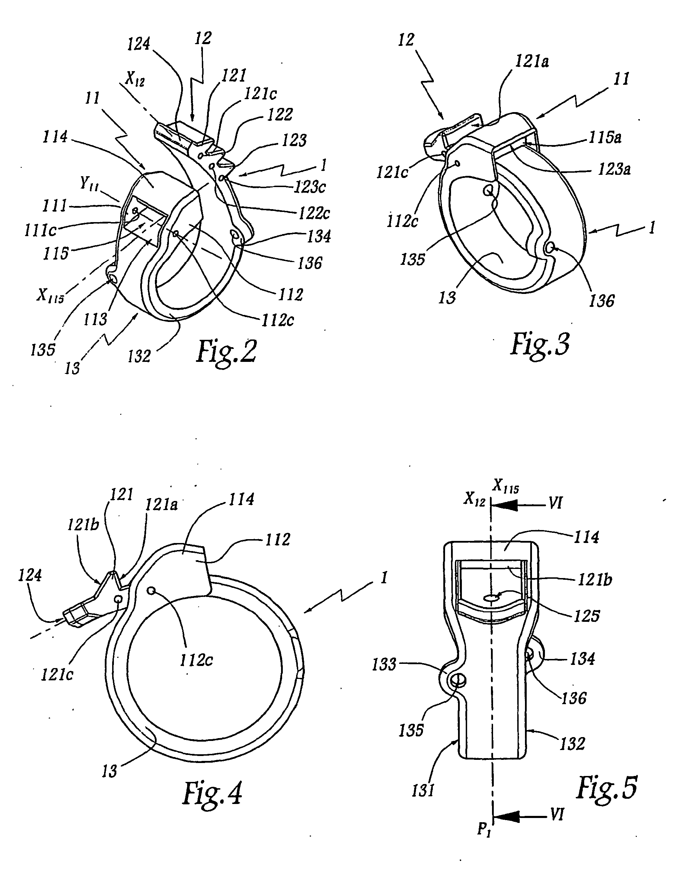

L'anneau 1 représenté sur les figures 1 à 9 est monobloc et moulé dans un matériau élastomère. L'anneau 1 présente une certaine élasticité qui lui permet d'être amené, à partir de sa position ouverte représentée à la figure 2, dans une position fermée représentée aux figures 1, 3 à 6 et 9 dans laquelle il présente une configuration de collier qui lui permet d'entourer ou d'enserrer une partie P de l'estomac E d'un patient obtenue par la création d'une découpe D dans cet estomac.

L'anneau 1 est globalement en forme de bande et comprend une première extrémité 11 et une deuxième extrémité 12 entre lesquelles est définie une partie intermédiaire 13 à section pleine dépourvue de partie dilatable et destinée à venir en appui et à entourer, par sa surface interne, la partie P.

L'extrémité 11 est pourvue de deux cloisons 111 qui s'étendent à partir du prolongement 113 de la partie 13 au niveau de l'extrémité 11 et qui sont reliées par un barreau 114. Il est ainsi défini entre les éléments 111 à 114 une ouverture 115 en forme de tunnel sur la longueur du barreau 114 pris parallèlement à un axe longitudinal X₁₁₅ de l'ouverture 115.

Sur sa face intérieure tournée vers le prolongement 113, le barreau 114 est pourvu d'une dent 116 définie entre une surface 116a globalement perpendiculaire à l'axe X₁₁₅ et une surface 116b inclinée par rapport à cet axe d'un angle α de l'ordre de 40°. En pratique l'angle α peut avoir une valeur comprise entre 30 et 60°. L'orientation de la surface 116b est telle qu'elle se rapproche du barreau 114 en se rapprochant du côté 115a d'entrée de l'ouverture 115 orientée à l'opposée de la portion 13.

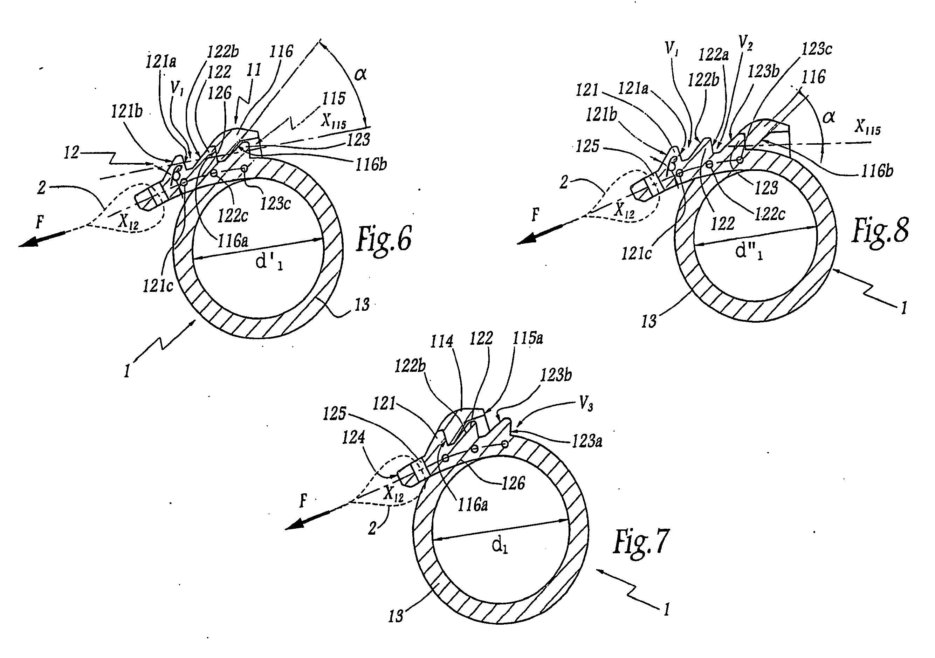

L'extrémité 12 s'étend dans le prolongement de la portion 13. On note X₁₂ son axe longitudinal qui est courbe dans le plan des figures 6 à 8 compte tenu du caractère courbe de l'extrémité 12. L'extrémité 12 porte trois dents 121, 122 et 123 ménagées respectivement sur le côté externe de l'extrémité 12 et se suivant l'une derrière l'autre, selon l'axe X₁₂, à partir du bord libre 124 de l'extrémité 12. La dent 121 est la plus proche du bord 124, alors que la dent 123 est la plus éloignée de ce bord.

La dent 121 est définie entre une surface 121a globalement perpendiculaire à l'axe X₁₂ et une surface 121b inclinée par rapport à cet axe d'un angle β de l'ordre de 40°. En pratique la valeur de l'angle β est égale à celle de l'angle α et comprise entre 30 et 60°. La surface 121b se rapproche d'une partie 126 de l'extrémité 12, à partir de laquelle s'étendent les dents 121 à 123 et qui prolonge la portion 13, en se rapprochant du bord 124.

Les dents 122 et 123 ont sensiblement la même géométrie que la dent 121 et sont également chacune définies entre une surface 122a ou 123a globalement perpendiculaire à l'axe X₁₂ et à une surface 122b ou 123b inclinée par rapport à cet axe du même angle β, dans le même sens que la surface 121b.

Les dents 121 et 122 définissent entre elles un volume V₁ de forme sensiblement complémentaire de la dent 116. De la même manière, les dents 122 et 123 définissent entre elles un volume V₂ de forme sensiblement complémentaire à la dent 116.

L'extrémité 12 est également pourvue d'un perçage 125 ménagé entre le bord libre 124 et la dent 121 et dans lequel peut être introduit un fil de suture 2 représenté en traits mixtes uniquement aux figures 6 à 8, ce fil de suture permettant d'exercer sur l'extrémité 12 un effort F de traction. En variante, le fil de suture peut être remplacé par d'autres moyens de traction de l'extrémité 12, par exemple une boucle formée d'un cathéter en silicone. On peut même prévoir que l'extrémité 12 soit moulée avec un anneau de traction intégré.

Ainsi, à partir de la configuration de la figure 2, il est possible d'introduire le bord 124 de l'extrémité 12 dans l'ouverture 115 par le côté 115a de cette ouverture visible à la figure 3 et opposé à la portion 13. En faisant traverser l'ouverture 115 au fil 2, il est possible de récupérer ce fil par le côté de l'ouverture 115 visible à la figure 2 puis de tirer sur l'extrémité 12 par l'effort F au moyen du fil 2, ce qui amène la surface 121b en appui contre le surface 116b. Compte tenu du sens d'inclinaison des surfaces 116b et 121b respectivement par rapport aux axes X₁₁₅ et X₁₂, un mouvement de glissement entre les dents 116 et 121 peut avoir lieu, moyennant une déformation élastique de certaines parties constitutives des extrémités 11 et 12, car le sens d'inclinaison des surfaces 116b et 121b est compatible avec la progression de l'extrémité 12 dans l'ouverture 115 sous l'effet de l'effort F.

Ceci permet de faire traverser complètement l'ouverture 15 à la dent 121 et d'amener l'extrémité 12 dans une première configuration de fermeture de l'anneau 1 qui est représentée à la figure 7. Dans cette configuration, le diamètre intérieur moyen de l'anneau 1 a une première valeur d₁. La dent 116 est alors engagée dans le volume V₁ où elle empêche tout retrait de l'extrémité 12 par rapport à l'ouverture 115, dans un sens opposé au déplacement précédemment effectué, par l'appui de sa surface 116a contre la surface 121a de la dent 121. Le fait que le volume V₁ est de forme sensiblement complémentaire de la dent 116 améliore encore l'effet de blocage obtenu.

Si le chirurgien le juge nécessaire, le maintien de l'effort F permet de déplacer plus avant l'extrémité 12 à l'intérieur de l'ouverture 115 pour atteindre la position des figures 3 à 6 où la dent 116 est engagée dans le volume V₂. Le diamètre intérieur de l'anneau 1 a alors une valeur d'₁ inférieure à la valeur d₁.

Si un diamètre moyen encore inférieur doit être atteint, il est possible de maintenir encore l'effort F pour engager encore plus profondément l'extrémité 112 dans l'ouverture 115 et atteindre la position de la figure 8 où la dent 116 est en appui, dans un volume V₃ jouxtant la dent 123 à l'opposé du bord 124, contre la surface 123a de la dent 123. Le diamètre intérieur de l'anneau 1 a alors une valeur d''₁ inférieure à la valeur d'₁.

Le diamètre moyen de l'anneau 1 est égal à la moyenne des diamètres du collier fermé globalement circulaire formée par la portion 13 et les extrémités 11 et 12 imbriquées en configuration fermée de l'anneau, étant entendu ici que ce collier n'est pas rigoureusement circulaire.

Ainsi, les dents 116 et 121 à 123 prévues respectivement sur les extrémités 11 et 12 permettent d'immobiliser l'extrémité 112 par rapport à l'ouverture 115 dans chacune des trois configurations représentées respectivement aux figures 6 à 8, ce qui permet d'ajuster le diamètre intérieur de l'anneau 1 en configuration fermée à ses conditions d'implantation.

Chacune des cloisons 111 et 112 est percée d'un orifice 111c, respectivement 112c,ces orifices étant alignés selon une direction Y₁₁ perpendiculaire à l'axe 115.

Par ailleurs, l'extrémité 111 est pourvue de trois perçages 121c, 122c et 123c ménagés respectivement au niveau des dents 121 à 123, dans la partie 126 de l'extrémité 12 qui prolonge la portion 13.

Les perçages 121c, 122c et 123c s'étendent selon des directions perpendiculaires à l'axe X₁₂ et sont disposés par rapport aux dents 121 et 122 de telle sorte que l'orifice 121c est aligné sur la direction Y₁₁ dans la configuration de la figure 7, alors que l'orifice 122c est aligné sur la direction Y₁₁ dans la configuration de la figure 6 et que l'orifice 123c est aligné sur la direction Y₁₁ dans la configuration de la figure 8.

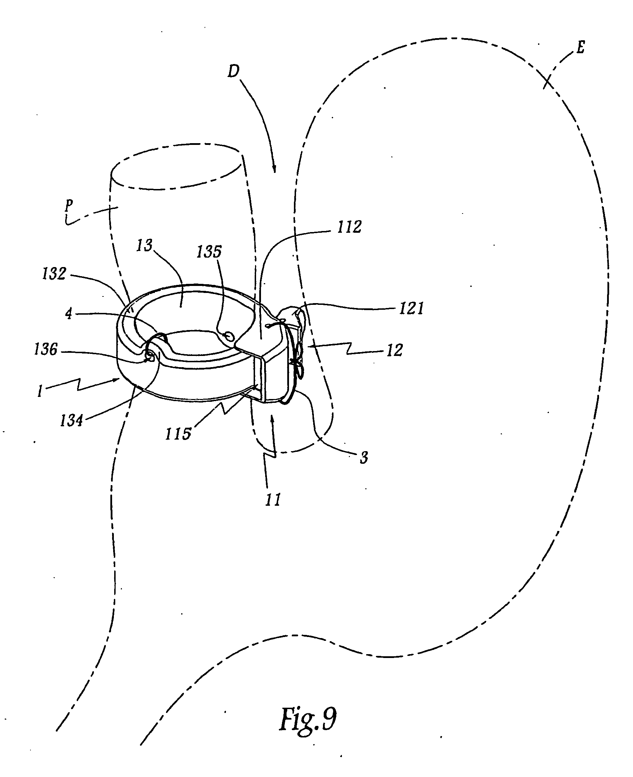

Ainsi, dans chacune des configurations représentées aux figures 6 à 8, l'un des perçages 121c, 122c et 123c forme avec les orifices 111c et 112c un canal dans lequel il est possible d'insérer un moyen de retenue ou de verrouillage des extrémités 11 et 12 dans la configuration correspondante. Par exemple, on insère dans le canal ainsi formé un fil de suture 3 visible uniquement aux figures 1 et 9 avant de nouer ce fil autour des extrémités 11 et 12 ainsi immobilisées et verrouillées l'une par rapport à l'autre.

En variante, à la place d'un fil de suture, on peut utiliser une agrafe ou tout autre moyen d'arrêt adapté et ne risquant pas de blesser la paroi gastrique.

Selon une variante non représentée de l'invention, un seul perçage pourrait être ménagé sur l'extrémité 12, alors que trois jeux d'orifices seraient ménagés dans les cloisons 111 et 112, le perçage de l'extrémité 12 étant amené en regard d'un des jeux d'orifices pour former un canal de passage d'un organe de retenue, selon la position de blocage sélectionnée.

Dans tous les cas, le ou les perçage(s) ménagé(s) dans l'extrémité 12 forme(nt) un ou des orifice(s) de passage d'un lien ou d'un organe de verrouillage des extrémités 11 et 12 l'une par rapport à l'autre.

On note P₁ le plan médian de l'anneau 1 qui est le plan selon lequel est effectué la coupe de la figure 6. Les axes X₁₁₅ et X₁₂ appartiennent à ce plan. La partie 13 est pourvue, sur chacun de ses bords longitudinaux 131 et 132 et de part et d'autre du plan P₁, d'une oreille 133, respectivement 134, dans laquelle est ménagé un orifice 135, respectivement 136, de passage d'un fil de ligature de l'anneau 1 sur la partie P de l'estomac. Ces orifices 135 et 136 sont sensiblement diamétralement opposés lorsque l'anneau est en configuration fermée. Ces orifices font saillie de part et d'autre du plan P₁ de sorte que, quelle que soit la voie d'abord utilisée pour la pose de l'anneau 1, l'un de ces orifices est accessible au chirurgien pour la pose d'une ligature 4 d'immobilisation sur la partie P.

Cette répartition des orifices 135 et 136 facilite le travail du chirurgien. En effet, selon le sens de pose de l'anneau 1 et comme représenté aux figures 1 et 9, si l'on suppose que le chirurgien aborde l'estomac E dans le sens où l'on observe ces figures, le chirurgien peut utiliser l'orifice 135 (figure 1) ou l'orifice 136 (figure 9) pour ligaturer l'anneau 1 à la partie P.

Il convient de noter que l'anneau 1 peut également être implanté avec ses extrémités 11 et 12 engagées dans la découpe D tout en ayant son orifice 135 accessible pour le chirurgien, comme dans la configuration de la figure 1.

L'invention a été décrite en référence à un anneau 1 avec lequel trois positions d'immobilisation de l'extrémité 12 dans l'extrémité 11 sont possibles. Elle est cependant applicable avec un anneau où seules deux positions d'immobilisation sont prévues ou, au contraire, plus de trois positions d'immobilisation sont prévues. Le nombre de dent de l'extrémité 12 est adapté au nombre de positions d'immobilisation que l'on cherche à obtenir.

L'invention a été représentée dans le cas d'un anneau utilisé pour une opération de gastroplastie verticale calibrée. Elle est cependant applicable à un anneau utilisé pour le calibrage d'une partie d'estomac, avant l'anastomose, dans le cadre d'un court-circuit gastrique, voire à un anneau utilisé pour calibrer le pylore d'un patient.

## Revendications

1. Anneau gastrique (1) dépourvu de partie dilatable, ledit anneau comprenant deux extrémités (11, 12) et, entre lesdites extrémités, une portion intermédiaire (13) à section pleine destinée à venir en appui simple sur une partie (P) d'un estomac (E) à entourer, une première extrémité (11) formant une ouverture (115) dans laquelle peut être introduite la deuxième extrémité (12), ledit anneau comprenant des reliefs (116, V₁-V₃) de blocage de ladite deuxième extrémité (12) dans ladite ouverture (115), dans au moins deux positions distinctes (figures 6 à 8), **caractérisé en ce qu'**il comprend, en plus desdits reliefs (116, V₁, V₂, V₃), des moyens (111c, 112c, 121c, 122c, 123c, 3) de verrouillage de ladite deuxième extrémité (12) dans ladite ouverture (115), dans chacune desdites positions, lesdits moyens de verrouillage comprenant deux premiers orifices (111c, 112c) ménagés dans deux cloisons (111, 112) appartenant à la première extrémité (11) et entre lesquelles est définie ladite ouverture (115) et au moins deux deuxièmes orifices (121c, 122c, 123c) ménagés dans l'autre extrémité (12), l'un desdits deuxièmes orifices étant aligné avec lesdits premiers orifices dans chacune desdites positions, lesdits orifices formant alors ensemble un canal de passage d'un organe (3) de retenue desdites extrémités l'une par rapport à l'autre et **en ce que** lesdits deux premiers orifices (111c, 112c) sont alignés selon une direction (Y₁₁) perpendiculaire à un axe longitudinal (X₁₁₅) de ladite ouverture.

2. Anneau selon la revendication 1, **caractérisé en ce que** ladite ouverture (115) est pourvue d'un relief (116) de blocage apte à coopérer, en fonction du diamètre sélectionné pour ledit anneau (1), avec l'un de plusieurs reliefs (V₁, V₂, V₃) de blocage complémentaires ménagés sur ladite deuxième extrémité (12).

3. Anneau selon la revendication 2, **caractérisé en ce que** ledit relief de blocage de ladite ouverture est une dent (116) faisant saillie à partir d'un bord (114) de ladite ouverture (115) en direction du bord opposé (113).

4. Anneau selon la revendication 2, **caractérisé en ce que** lesdits reliefs complémentaires (V₁, V₂, V₃) ménagés sur ladite deuxième extrémité (12) sont formés par des dents (121, 122, 123) disposées l'une derrière l'autre selon un axe longitudinal (X₁₂) de ladite deuxième extrémité.

5. Anneau selon la revendication 4, **caractérisé en ce que** lesdits deuxièmes orifices (121c, 122c, 123c) sont ménagés respectivement au niveau desdites dents (121, 122, 123) de ladite deuxième extrémité (12), dans une partie (126) de ladite deuxième extrémité (12) qui prolonge ladite portion intermédiaire (13), et **en ce que** lesdits orifices s'étendent selon des directions perpendiculaires à un axe longitudinal (X₁₂) de ladite seconde extrémité.

6. Anneau selon là revendication 4, **caractérisé en ce que** lesdites dents (116, 121, 122, 123) ont des surfaces (116b, 121b, 122b, 123b) inclinées, respectivement par rapport à un axe médian (X₁₁₅) de ladite ouverture (115) et par rapport à l'axe longitudinal (X₁₂) de ladite deuxième extrémité (12), dans un sens compatible avec le déplacement de ladite deuxième extrémité à travers ladite ouverture correspondant à un resserrement de l'anneau.

7. Anneau selon la revendication 6, **caractérisé en ce que** lesdites dents (116, 121, 122, 123) ont des surfaces (116a, 121a, 122a, 123a) perpendiculaires respectivement audit axe médian (X₁₁₅) de ladite ouverture (115) et audit axe longitudinal (X₁₂) de ladite deuxième extrémité (12), lesdites surfaces étant en appui l'une contre l'autre lorsque ladite seconde extrémité est immobilisée dans ladite ouverture dans l'une desdites positions.

8. Anneau selon l'une des revendications 3 à 7, **caractérisé en ce qu'**un volume (V₁, V₂) défini entre deux dents adjacentes (121, 122, 123) ménagées sur ladite deuxième extrémité (12) est sensiblement complémentaire de la dent (116) qui fait saillie à partir d'un bord (114) de ladite ouverture (115).

9. Anneau selon l'une des revendications précédentes, **caractérisé en ce que** ladite portion intermédiaire (13) est pourvue, sur chacun de ses bords longitudinaux (131, 132), d'un orifice (135, 136) de passage d'un fil de ligature (4), lesdits orifices étant sensiblement diamétralement opposés lorsque ladite deuxième extrémité (12) est immobilisée dans ladite ouverture (115) dans l'une desdites positions.

## Patentansprüche

1. Magenring (1) ohne dehnbare Bereiche, wobei der Ring zwei Enden (11, 12) und zwischen den Enden einen Zwischenbereich (13) mit geschlossenem Querschnitt umfasst, der vorgesehen ist, in Flächenauflage auf einem zu umgreifenden Teil (P) eines Magens (E) zu kommen, wobei ein erstes Ende (11) eine Öffnung (115) bildet, in die das zweite Ende (12) eingeführt werden kann, wobei der Ring Reliefs (116, V₁-V₃) zur Blockierung des zweiten Endes (12) in der Öffnung (115) in mindestens zwei unterschiedlichen Positionen (Figuren 6 bis 8) aufweist, **dadurch gekennzeichnet, dass** er zusätzlich zu den Reliefs (116, V₁, V₂, V₃) Mittel (111c, 112c, 121c, 122c, 123c, 3) zur Verriegelung des zweiten Endes (12) in der Öffnung (115) in jeder der Positionen umfasst, wobei die Mittel zur Verriegelung zwei erste Öffnungen (111c, 112c), die in zwei zu dem ersten Ende (11) gehörenden Trennwänden (111, 112) eingearbeitet sind, zwischen denen die Öffnung (115) begrenzt ist, und mindestens zwei zweite Öffnungen (121c, 122c, 123c), die in dem
anderen Ende (12) eingearbeitet sind, umfasst, wobei eine der zwei zweiten Öffnungen mit den ersten Öffnungen in jeder der Positionen ausgerichtet ist und die Öffnungen somit zusammen einen Durchgangskanal für ein Element (3) zum Zusammenhalten der zwei Enden zueinander bilden, und dass die zwei ersten Öffnungen (111c, 112c) gemäß einer Richtung (Y₁₁) senkrecht zu einer Längsachse (X₁₁₅) der Öffnung ausgerichtet sind.

2. Ring nach Anspruch 1, **dadurch gekennzeichnet, dass** die Öffnung (115) mit einem Blockierungsrelief (116) versehen ist, das geeignet ist, abhängig vom für den Ring (1) gewählten Durchmesser mit einem von mehreren komplementären Blockierungsreliefs (V₁, V₂, V₃) zusammenzuarbeiten, die auf dem zweiten Ende (12) angeordnet sind.

3. Ring nach Anspruch 2, **dadurch gekennzeichnet, dass** das Blockierungsrelief der Öffnung ein Zahn (116) ist, der von einem Rand (114) der Öffnung (115) in Richtung des gegenüberliegenden Randes (113) hervorspringt.

4. Ring nach Anspruch 2, **dadurch gekennzeichnet, dass** die auf dem zweiten Ende (12) angeordneten komplementären Reliefs (V₁, V₂, V₃) durch Zähne (121, 122, 123) gebildet werden, die, einer hinter dem anderen, gemäß einer Längsachse (X₁₂) des zweiten Endes angeordnet sind.

5. Ring nach Anspruch 4, **dadurch gekennzeichnet, dass** die zweiten Öffnungen (121c, 122c, 123c) jeweils in der Nähe der Zähne (121, 122, 123) des zweiten Endes (12) in einem Bereich (126) des zweiten Endes (12), der den Zwischenbereich (13) verlängert, eingearbeitet sind und dass die Öffnungen sich gemäß Richtungen erstrecken, die senkrecht zu einer Längsachse (X₁₂) des zweiten Endes liegen.

6. Ring nach Anspruch 4, **dadurch gekennzeichnet, dass** die Zähne (116, 121, 122, 123) Flächen (116a, 121a, 122a, 123a) aufweisen, die jeweils in Bezug auf eine Mittelachse (X₁₁₅) der Öffnung (115) und in Bezug auf die Längsachse (X₁₂) des zweiten Endes (12) in eine Richtung geneigt sind, die kompatibel mit der Verschiebung des zweiten Endes durch die korrespondierende Öffnung bei einem Zusammenziehen des Ringes ist.

7. Ring nach Anspruch 6, **dadurch gekennzeichnet, dass** die Zähne (116, 121, 122, 123) Flächen (116a, 121a, 122a, 123a) aufweisen, die jeweils senkrecht zu der Mittelachse (X₁₁₅) der Öffnung (115) und zur Längsachse (X₁₂) des zweiten Endes (12) sind, wobei die Flächen eine gegen die andere in Auflage sind, wenn das zweite Ende in der Öffnung in einer der Positionen festgelegt ist.

8. Ring nach einem der Ansprüche 3 bis 7, **dadurch gekennzeichnet, dass** ein zwischen zwei benachbarten Zähnen (121, 122, 123), die auf dem zweiten Ende (12) angeordnet sind, definiertes Volumen (V₁, V₂) im Wesentlichen komplementär zu dem Zahn (116) ist, der von dem Rand (114) der Öffnung (115) hervorspringt.

9. Ring nach einem der vorhergehenden Ansprüche, **dadurch gekennzeichnet, dass** der Zwischenbereich (13) auf jedem seiner Längsränder (131,132) mit einer Öffnung (135,136) für den Durchgang eines Bindefadens (4) versehen ist, wobei die Öffnungen im Wesentlichen diametral gegenüberliegend sind, wenn das zweite Ende (12) in der Öffnung (115) in einer der Positionen festgelegt ist.

## Claims

1. A gastric ring (1) having no expandable portion, said ring having two ends (11, 12) and, between said ends, an intermediate portion (13) of solid section for bearing simply against a portion (P) of a stomach (E) to be surrounded, a first end (11) forming an opening (115) in which it is possible to insert the second end (12), said ring including portions in relief (116, V₁-V₃) for blocking said second end (12) in said opening (115) in at least two distinct positions (Figures 6 to 8), the ring being **characterized in that** it comprises, in addition to said portions in relief (116, V₁, V₂, V₃), means (111c, 112c, 121c, 122c, 123c, 3) for locking said second end (12) in said opening (115) in each of said positions, said locking means comprising two first orifices (111c, 112c) formed in two partitions (111, 112) belonging to the first end (11) and between which said opening (115) is defined, and at least two second orifices (121c, 122c, 123c) formed in the other end (12), one of said second orifices being in alignment with said first orifice in each of said positions, said orifices then together forming a channel for passing a holding member (3) for holding said ends relative to each other and **in that** said two second orifices (111c, 112c) are in alignment on a direction (Y₁₁) perpendicular to a longitudinal axis (X₁₁₅) of said opening.

2. A ring according to claim 1, **characterized in that** said opening (115) is provided with a blocking portion in relief (116) suitable for co-operating, as a function of the diameter selected for said ring (1), with one of a plurality of complementary blocking portions in relief (V₁, V₂, V₃) formed on said second end (12).

3. A ring according to claim 2, **characterized in that** said blocking portion in relief of said opening is a tooth (116) projecting from an edge (114) of said opening (115) towards the opposite edge (113).

4. A ring according to claim 2, **characterized in that** said complementary portions in relief (V₁, V₂, V₃) formed on said second end (12) are formed by teeth (121, 122, 123) disposed one behind another along a longitudinal axis (X₁₂) of said second end.

5. A ring according to claim 4, **characterized in that** said second orifices (121c, 122c, 123c) are formed respectively at the level of said teeth (121, 122, 123) of said second end (12), in a portion (126) of said second end (12) that extends said intermediate portion (13), and **in that** said orifices extend in directions that are perpendicular to a longitudinal axis (X₁₂) of said second end.

6. A ring according to claim 4, **characterized in that** said teeth (116, 121, 122, 123) have inclined surfaces (116b, 121b, 122b, 123b) that are inclined, respectively relative to a middle axis (X₁₁₅) of said opening (115) and relative to the longitudinal axis (X₁₂) of said second end (12), in directions that are compatible with said second end moving through said opening in a direction compatible with tightening the ring.

7. A ring according to claim 6, **characterized in that** said teeth (116, 121, 122, 123) have respective surfaces (116a, 121a, 122a, 123a) that are perpendicular to said middle axis (X₁₁₅) of said opening (115) and to said longitudinal axis (X₁₂) of said second end (12), said surfaces bearing one against another when said second end is prevented from moving in said opening in one of said positions.

8. A ring according to any one of claims 3 to 7, **characterized in that** a volume (V₁, V₂) defined between two adjacent teeth (121, 122, 123) formed on said second end (12) is substantially complementary to the tooth (116) that projects from an edge (114) of said opening (115).

9. A ring according to any preceding claim, **characterized in that** said intermediate portion (13) is provided, on each of its longitudinal edges (131, 132), with an orifice (135, 136) for passing a ligating suture (4), said orifices being substantially diametrically opposite when said second end (12) is prevented from moving in said opening (115) in one of said positions.
